Europäisches Patentamt

European Patent Office

Office européen des brevets

(11) Publication number: **0 422 490 A2**

# EUROPEAN PATENT APPLICATION

(21) Application number: 90118923.3

(22) Date of filing: 04.10.90

(51) Int. Cl.5: **A61K 31/23**, A23D 7/00, A23D 9/00, A23L 1/30, A23L 1/03

(30) Priority: **12.10.89 US 420197**

(43) Date of publication of application:
**17.04.91 Bulletin 91/16**

(84) Designated Contracting States:
**AT BE CH DE DK ES FR GB GR IT LI LU NL SE**

(71) Applicant: **THE PROCTER & GAMBLE COMPANY**
**One Procter & Gamble Plaza**
**Cincinnati Ohio 45202(US)**

(72) Inventor: **Jandacek, Ronald James**
**125 Junefield Avenue**
**Cincinnati, Ohio 45218(US)**
Inventor: **Yang, David Kee**
**6881 Fox Hill Lane**
**Cincinnati, Ohio 45236(US)**
Inventor: **Hemingway, Ronald Lee**
**7405 Thumbelina Lane**
**Cincinnati, Ohio 45242(US)**
Inventor: **Guttag, Eric Ward**
**1110 Springfield Pike, Unit 6**
**Cincinnati, Ohio 45215(US)**

(74) Representative: **Suslic, Lydia et al**
**Procter & Gamble European Technical**
**Center N.V. Temselaan 100**
**B-1853 Strombeek-Bever(BE)**

(54) Triglycerides containing saturated fatty acids having 20 to 24 carbon atoms useful in lowering blood cholesterol levels.

(57) Triglyceride fats based on very long chain $C_{20}$ to $C_{24}$ saturated fatty acid triglycerides that are effective in lowering blood cholesterol levels are disclosed. These triglyceride fats are useful in pharmaceutical compositions in effective unit dosage amounts for inhibiting cholesterol absorption and in methods for treating hypercholesterolemia. These triglyceride fats are also useful, in whole or in part, in the triglyceride fat component of fat-containing food compositions. These fat-containing food compositions can be used as part of a dietary method or regimen for lowering blood cholesterol levels.

# TRIGLYCERIDES CONTAINING SATURATED FATTY ACIDS HAVING 20 TO 24 CARBON ATOMS USEFUL IN LOWERING BLOOD CHOLESTEROL LEVELS

## Technical Field

This application relates to triglycerides containing saturated fatty acids having 20 to 24 carbon atoms that are useful in lowering blood cholesterol levels. This application particularly relates to food compositions, methods for lowering cholesterol, pharmaceutical compositions in unit dosage form, and methods for treating hypercholesterolemia involving the use of these triglycerides.

Elevated blood cholesterol is recognized as being a risk factor in cardiovascular disease and comprises a major health care problem today. Epidemiological studies have demonstrated that, with few exceptions, populations consuming large quantities of saturated fat and cholesterol have relatively high concentrations of blood cholesterol. While it is recognized that other factors can contribute to the development of cardiovascular disease, there appears to be a causal relationship between the concentration of cholesterol in the blood and the incidence of coronary disease. In particular, high blood cholesterol levels (hypercholesterolemia) can result in the accumulation of undesirable amounts of cholesterol in various parts of the circulatory system (atherosclerosis) or in the soft tissues (xanthomatosis) leading to coronary disease, and potentially coronary mortality.

The relationship between cholesterol and the incidence of coronary disease is not determined solely by the total level of cholesterol in the blood. It is now known that cholesterol can be present in the bloodstream in essentially two different forms. One form referred to as high density lipoprotein (HDL) cholesterol is actually considered to be "good." It is only the low density lipoprotein (LDL) form of cholesterol that significantly contributes to coronary disease.

There have been essentially two dietary approaches to lowering blood cholesterol levels, especially the LDL form of cholesterol, in humans. The first of these approaches is referred to hereafter as the "dietary fat" approach. The dietary fat approach typically involves the use of a "low fat" diet as a means for lowering the total blood cholesterol level, thereby indirectly lowering the level of undesirable LDL cholesterol. Also, the dietary fat approach focuses on the particular fats that are consumed. Conventional wisdom has been that the saturated fats, particularly those high in long chain saturated fatty acids (e.g. stearic and especially palmitic acid) should be avoided because these fatty acids "generate" cholesterol in the body. Fats which are high in noncholesterol-generating fatty acids are consumed instead to effectively lower total blood cholesterol levels. Examples of such fats are those high in polyunsaturated fatty acids, in particular vegetable oils, such as soybean oil, safflower oil and sunflower oil.

There is not total agreement that all long chain saturated fatty acids contribute to higher blood cholesterol levels. Bonanome et al, "Effect of Dietary Stearic Acid on Plasma Cholesterol in Lipoprotein Levels," New Eng. J. Med. , Vol. 318 (1988), pp. 1244-48, discloses a study in humans on the metabolic effects of consuming diets high in palmitic acid (palm oil), high in stearic acid (completely hydrogenated soybean oil), or high in oleic acid (high oleic safflower oil). Compared to the high palmitic acid diets, Bonanome et al found that plasma total cholesterol levels decreased during consumption of the high stearic acid and high oleic acid diets. Similarly, LDL cholesterol levels fell during consumption of these diets. Based on these findings, Bonanome et al concluded that "all saturated fatty acids do not have the same cholesterol-raising potential." (The particular mechanism by which stearic acid causes this cholesterol-lowering effect appears to be similar to that of the polyunsaturated fatty acids, i.e. stearic acid does not "generate" cholesterol in the body.) See also Grand et al, "Comparison of the Effects of Palmitic and Stearic Acids in the Diet on Serum Cholesterol in Men," Am. J. Clin. Nutr. , Vol. 23 (1970), pp. 1184-93, which describe a study involving cocoa butter, cocoa butter substitutes, natural palm oil and synthetic palm oil which suggests that stearic acid has no effect on blood cholesterol concentrations in men relative to palmitic acid.

The other dietary approach to lowering blood cholesterol levels involves limiting dietary cholesterol absorption or consumption. One significant example of this approach is hereafter referred to as the "cholesterol absorption inhibition" method. The cholesterol absorption inhibition method typically involves the use of an agent that effectively inhibits the absorption by the body of cholesterol that is already present in the diet. One example of a cholesterol absorption inhibiting agent are certain nondigestible, nonabsorbable polyol fatty acid polyesters, particularly sucrose polyesters. See, for example, U.S. Patent 3,954,976 to Mattson et al, issued May 4, 1976. Other agents that have been found to inhibit the absorption of dietary cholesterol include ion exchange resins such as Colestipol or Questran, plant sterols such as $\beta$-sitosterol,

linoleoylamide and certain antibiotics.

These cholesterol absorption inhibiting agents operate by a variety of mechanisms. For example, the nondigestible/nonabsorbable polyol fatty acid polyesters act as a "oil sink" for the cholesterol, thereby preventing its absorption by the intestinal wall. Nonabsorbable anion exchange resins act by binding the cholesterol-rich bile salts and thereby block the enterohepatic flux of cholesterol. Antibiotics, such as Neomycin, exhibit a digitonin-like cholesterol precipitating effect in the gastrointestinal tract.

All of these dietary approaches, by themselves, have drawbacks. For example, the dietary fat approach merely prevents the generation of additional cholesterol, but does not prevent the absorption of cholesterol already present in the diet. Conversely, the cholesterol absorption inhibition method, while effective on cholesterol already present in the diet, has no effect on fats in the diet which can generate cholesterol in the body. In addition, unlike the dietary fat approach, the cholesterol absorption inhibition method, by itself, provides no nutritional value to the person. Accordingly, it would be desirable to be able to combine both the dietary fat and cholesterol absorption inhibition approaches to reduce total blood cholesterol levels, in particular LDL cholesterol levels. It would especially be desirable to combine these approaches by utilizing the same material or agent.

## Background Art

### A. Food Compositions Containing Substantial Levels of Behenic Acid-Containing Triglycerides

Japanese Laid-Open Patent Application 63/22133 to Maeda et al, published January 29, 1988, discloses "roll-in" oil/fat compositions for pastries containing 15-70% mixed acid group triglycerides containing 15-70% $C_{20}$-$C_{24}$ saturated fatty acids and 20-60% $C_{16}$-$C_{22}$ unsaturated fatty acids. The Examples disclose mixed triglycerides containing from about 45 to 60% $C_{20}$-$C_{24}$ saturated fatty acids that are obtained by interesterification of 50:50 blends of behenic acid triglycerides (or hardened high erucic rapeseed oil) with safflower oil (or olive oil). The oil/fat compositions based on these mixed triglycerides can comprise from about 9 to about 33% $C_{20}$-$C_{24}$ saturated fatty acids. See also published European patent application 268,431 to Nakano et al, published May 25, 1988, which discloses similar mixed triglycerides useful as migration inhibitors for fats and oils used in cakes.

U.S. Patent 4,705,692 to Tanaka et al, issued Nov. 10, 1987, discloses a cocoa butter substitute comprising 40 to 95% of an oleaginous component containing at least 70% cocoa butter-type triglycerides (i.e. 1,3-disaturated-2-oleoyl glycerides) and 5 to 60% of an oleaginous component containing 40 to 100% mixed acid triglycerides like those disclosed in Japanese Laid-Open patent application 63/22133. For the cocoa butter substitutes specifically disclosed as being useful, the level of $C_{20}$-$C_{24}$ saturated fatty acids can range as high as about 27%. In some of the comparative cocoa butter substitute examples, the level of $C_{20}$-$C_{24}$ saturated fatty acids can range as high as about 60%. See also U.S. Patent 4,726,959 to Momura et al, issued February 23, 1988, which discloses similar mixed acid triglycerides used as fat bloom inhibitors for hard butters.

### B. The Cholesterol-Lowering Effect of Triglycerides Containing Stearic Acid

Bonanome et al, "Effect Dietary Stearic Acid on Plasma Cholesterol and Lipoprotein Levels," New Eng. J. Med. , Vol. 318 (1988), pp. 1244-48, discloses a study in humans of the metabolic effects of consuming diets high in palmitic acid (palm oil), high in stearic acid (completely hydrogenated soybean oil), or high in oleic acid (high oleic safflower oil). Compared to the high palmitic acid diet, plasma total cholesterol decreased by an average of 14% during consumption of the high stearic acid diet and by 10% during consumption of the high oleic acid diet. Low density lipoprotein (LDL) cholesterol levels fell by 21% during consumption of the high stearic acid diet and by 15% during consumption of the high oleic acid diet. From these results, it was concluded that "all saturated fatty acids do not have the same cholesterol-raising potential." See also Grande et al, "Comparison of the Effects of Palmitic and Stearic Acids in the Diet on Serum Cholesterol in Men," Am. J. Clin. Nutr. , Vol. 23 (1970), pp. 1184-93, which describes a study involving cocoa butter, cocoa butter substitutes, natural palm oil and synthetic palm oil which suggests that stearic acid has no effect on serum cholesterol concentration in man relative to palmitic acid.

EP 0 422 490 A2

C. The Absorbability of Behenic-Acid Containing Triglycerides

Mattson and Streck, "Effect of the Consumption of Triglycerides Containing Behenic Acid on the Lipid Content of the Heart of Weanling Rats," J. Nutr. , Vol. 104 (1974), pp. 483-88, discloses a study involving weanling rats fed diets containing 19% fat that consisted of various levels of glycerides of behenic or erucic acid. Consumption of the erucic acid-containing fat increased heart lipids two to three-fold. By contrast, consumption of the behenic acid-containing fat resulted in no accumulation of lipids in the heart. A similar lack of increase in heart lipids was observed when the dietary fat was 2-behenoyl dilinolein. This lack of increase in heart lipids was determined to be due to impaired absorption of the behenic acid. See also Nolen, "Biological Evaluation of Hydrogenated Rapeseed Oil," J. Am. Oil Chem. Soc. , Vol. 58 (1981), pp. 31-37, which discloses a 90 day rat-feeding study where the absorption of behenic acid from two hydrogenated rapeseed oils was found to be only 12% and 17%, respectively.

D. The Ability of Nondigestible Nonabsorbable Polyol Polyesters (e.g., Sucrose Polyesters) to Inhibit the Absorption of Cholesterol

U.S. Patent 3,954,976 to Mattson et al, issued May 4, 1976, discloses pharmaceutical compositions for inhibiting the absorption of cholesterol which comprise an effective unit dosage of a nondigestible, nonabsorbable, polyol fatty acid polyester, preferably a sucrose polyester. Also disclosed are methods for treating and/or preventing hypercholesterolemia by administering successive therapeutically effective doses of these polyol polyesters. See also U.S. Patent 4,005,195 to Jandacek, issued January 25, 1977 (combinations of liquid polyol polyesters and anti-anal leakage agents useful in inhibiting the absorption of cholesterol and in treating and/or preventing hypercholesterolemia); U.S. Patent 4,005,196 to Jandacek et al, issued January 25, 1977 (combinations of liquid polyol polyesters, anti-anal leakage agents and fat-soluble vitamins useful in inhibiting the absorption of cholesterol and in treating and/or preventing hypercholesterolemia); U.S. Patent 4,034,083 to Mattson, issued July 5, 1977 (combination of polyol polyesters and fat-soluble vitamins useful in inhibiting the absorption of cholesterol and in treating and/or preventing hypercholesterolemia).

DISCLOSURE OF THE INVENTION

The present invention relates to the use of triglycerides which have very long chain $C_{20}$ to $C_{24}$ saturated fatty acids. Surprisingly, these very long chain fatty acid triglycerides have been found to effectively inhibit the absorption of cholesterol that is already present in the diet. In addition, the $C_{20}$ to $C_{24}$ saturated fatty acids present in these triglycerides do not, themselves, "generate" cholesterol in the body.

The present invention particularly relates to triglyceride fats based on these very long chain fatty acid triglycerides, which have fatty compositions comprising from about 5 to about 70% $C_{20}$ to $C_{24}$ saturated fatty acids. These triglyceride fats are useful in pharmaceutical compositions in effective unit dosage amounts of from about 1 to about 20 grams for inhibiting the absorption of cholesterol. These triglyceride fats are also useful in methods for treating hypercholesterolemia by systemically administering to an animal susceptible to or afflicted with hypercholesterolemia successively therapeutically effective doses of these fats.

The present invention further relates to the use of these triglyceride fats, in whole or in part, in the triglyceride fat component of fat-containing food compositions. One such triglyceride fat comprises:

(A) from about 10 to about 90% combined MLM, MML, LLM and LML triglycerides, wherein M is selected from the group consisting of $C_6$ to $C_{10}$ saturated fatty acids and mixtures thereof, and L is selected from the group consisting of $C_{20}$ to $C_{24}$ saturated fatty acids and mixtures thereof; and

(B) from about 10 to about 90% combined USS and SUS triglycerides, wherein U is selected from the group consisting of $C_{18}$ to $C_{24}$ unsaturated fatty acids and mixtures thereof, one S is selected from the group consisting of $C_{20}$ to $C_{24}$ saturated fatty acids and mixtures thereof, and the other S is selected from the group consisting of $C_{18}$ to $C_{24}$ saturated fatty acids and mixtures thereof.

These fat-containing food compositions, when ingested in a cholesterol-lowering amount, can be used as part of a dietary method or regimen for lowering blood cholesterol levels.

All weights, ratios and percentages used herein are on a weight basis unless otherwise specified.

4

## A. Very Long Chain Fatty Acid Triglycerides and Their Sources

The present invention is based on the discovery that certain very long chain fatty acid triglycerides are effective in inhibiting the absorption of cholesterol. These very long chain fatty acid triglycerides are particularly characterized by the fact that they contain one or more $C_{20}$ to $C_{24}$ saturated fatty acids. In addition to imparting cholesterol-inhibiting properties to the very long chain fatty acid triglycerides, these $C_{20}$ to $C_{24}$ saturated fatty acids do not, themselves, "generate" cholesterol in the body. Accordingly, these very long chain fatty acid triglycerides effectively combine the two dietary approaches to lowering blood cholesterol levels.

The particular mechanism by which these very long chain fatty acid triglycerides inhibit the absorption of cholesterol is not completely understood. One postulated mechanism is that the cholesterol is solubilized by these triglycerides. In other words, these triglycerides, and particularly the fats that contain them, act as a "oil sink" for the cholesterol. These triglycerides are also not very well absorbed due to the poor digestibility and absorptivity of the $C_{20}$ to $C_{24}$ saturated fatty acids they contain. As a result, any cholesterol solubilized by these triglycerides is effectively prevented from being absorbed by the intestinal wall.

Another postulated mechanism is based on the formation of esters between cholesterol and these $C_{20}$ to $C_{24}$ saturated fatty acids. As noted above, the $C_{20}$ to $C_{24}$ saturated fatty acids present in these triglycerides are poorly digested. However, some of these $C_{20}$ to $C_{24}$ saturated fatty acids are freed during the digestion of the very long chain fatty acid triglycerides in the gut. These free $C_{20}$ to $C_{24}$ fatty acids can then form esters with cholesterol (e.g., cholesteryl behenate) that are not readily absorbable by the intestinal wall. It is believed that both of these postulated mechanisms are operative to inhibit the absorption of cholesterol.

There are a number of edible fat/oil sources that contain substantial levels of these triglycerides having $C_{20}$ to $C_{24}$ saturated fatty acids. One source is high erucic acid rapeseed oil which has been hydrogenated (hardened) to an iodine value of about 10 or less. See, for example, U.S Patent 3,129,102 to Sanders, issued April 14, 1964, which is incorporated by reference. Another potential source of such triglycerides is fractionally crystallized and hardened peanut oil. See U.S. Patent 4,288,378 to Japikse et al, issued September 8, 1981 (herein incorporated by reference), where these very long chain fatty acid triglycerides can be obtained by randomly interesterifying a mixture of hydrogenated and unhydrogenated peanut oil that is fractionally crystallized to enrich the level of $C_{20}$ to $C_{24}$ fatty acid triglycerides, followed by substantially complete hydrogenation. Other fat and oil sources high in triglycerides having $C_{20}$ to $C_{24}$ saturated fatty acids can be derived from completely hardened marine oils such as herring oil and menhaden oil, and completely hardened meadow foam (Lymnanthes alba) sold by the Oregon Meadow Foam Growers Association, Salem, Oregon.

One preferred source of triglycerides having $C_{20}$ to $C_{24}$ saturated fatty acids are reduced calorie fats characterized by substantial levels of triglycerides selected from MLM, MML, LLM, and LML triglycerides, wherein M is a medium chain $C_6$ to $C_{10}$ saturated fatty acid residue and L is a long chain $C_{20}$ to $C_{24}$ saturated fatty acid residue. See U.S. application entitled "Reduced Calorie Fats Made from Triglycerides Containing Medium and Long Chain Fatty Acids," to Paul Seiden, Serial No. 329,620 (P&G Case 3760R), filed March 28, 1989 (herein incorporated by reference), which discloses reduced calorie fats useful as sources of very long chain fatty acid triglycerides for the present invention, and especially Examples 1 and 2 for methods for making same.

In these reduced calorie fats, "MML" represents a triglyceride containing a very long chain saturated acid residue in the #1 or #3 position (an end position) with two medium chain saturated fatty acid residues in the remaining two positions, while "MLM" represents a triglyceride with a very long chain fatty acid residue in the #2 position (the middle position) and two medium chain fatty acid residues in the #1 and #3 positions. (Typically, these MLM and MML triglycerides have carbon numbers predominantly in the range of from $C_{38}$ to $C_{42}$). Similarly, "LLM" represents a triglyceride with a medium chain fatty acid residue in the #1 or #3 position and two very long chain fatty acid residues in the remaining two positions, while "LML" represents a triglyceride with a medium chain fatty acid residue in the #2 position and two very long chain fatty acid residues in the #1 and #3 positions. (Typically, these LLM and LML triglycerides have carbon numbers predominantly in the range of $C_{52}$ to $C_{54}$.)

These reduced calorie fats can comprise at least about 30% combined MML, MLM, LLM and LML triglycerides, more preferably at least about 50% of these triglycerides, and most preferably at least about 80% of these triglycerides. Preferred reduced calorie fats comprise at least about 10% by weight of a mixture of MML and MLM triglycerides, more preferably at least about 35% of such combined triglycerides, and most preferably at least about 70% of such combined triglycerides. Preferred reduced calorie fats also comprise not more than about 90% combined LLM and LML triglycerides, more preferably not more than about 65% LLM and LML triglycerides, and most preferably not more than about 30% combined LLM and LML triglycerides. For most uses, these preferred reduced calorie fats also comprise minimized levels of MMM triglycerides and LLL triglycerides. By "MMM," as used herein, is meant a triglyceride containing medium chain saturated fatty acid residues at all three positions. Similarly, "LLL" represents a triglyceride containing very long chain saturated fatty acid residues at all three positions. These preferred reduced calorie fats comprise not more than about 15%, more preferably not more than about 10%, and most preferably not more than about 5% MMM triglycerides. These preferred reduced calorie fats also comprise not more than about 5%, more preferably not more than about 2%, and most preferably not more than about 1% by weight LLL triglycerides.

For optimum taste and other desired physical properties, certain of these reduced calorie fats have particularly preferred triglyceride compositions. These particularly preferred reduced calorie fats comprise at least about 85% of a mixture of MML and MLM triglycerides, more preferably at least about 90% of such combined triglycerides, and most preferably at least about 94% of such combined triglycerides. These preferred reduced calorie fats also comprise no more than about 5% combined LLM and LML triglycerides, more preferably no more than about 2% LLM and LML triglycerides, and most preferably no more than about 1% combined LLM and LML triglycerides. These particularly preferred reduced calorie fats further comprise no more than about 4%, preferably no more than about 2% and most preferably no more than about 1% MMM triglycerides, and no more than about 2%, preferably no more than about 1% and most preferably no more than about 0.5% LLL triglycerides.

These particularly preferred reduced calorie fats also have the following preferred and most preferred carbon number profiles (CNP):

| CNP | PREFERRED (%) | MOST PREFERRED (%) |
|---|---|---|
| 32 or lower | < 3 | < 1 |
| 34 | < 2 | < 1 |
| 36 | < 4 | < 2 |
| 38 | 15-40 | 15-30 |
| 40 | 35-60 | 45-55 |
| 42 | 15-35 | 20-30 |
| 44 | < 2 | < 1 |
| 46 | < 1 | < 0.6 |
| 48 | < 0.8 | < 0.6 |
| 50 | < 0.6 | < 0.5 |
| 52 | < 0.4 | < 0.3 |
| 54 or higher | < 0.9 | < 0.4 |

These reduced calorie fats are further characterized by particular fatty acid compositions. One important aspect of these fatty acid compositions is the total amount of medium chain $C_6$ to $C_{10}$ saturated fatty acids. These medium chain fatty acids generally control the melting point of the respective triglyceride mixture. In addition, these medium chain fatty acids are readily hydrolyzed (especially if attached at the #1 or #3 positions) by pancreatic lipase and then absorbed to provide a rapid energy source. However, these medium chain fatty acids, when metabolized, provide less total calories than longer chain fatty acids.

The fatty acid composition of these reduced calorie fats can comprise from about 15 to about 70%, preferably from about 40 to about 60%, and most preferably from about 45 to about 55% combined $C_6$ to $C_{10}$ saturated fatty acids. Due to the sources of medium chain saturated fatty acids typically used to synthesize these reduced calorie fats, the $C_8$ and $C_{10}$ saturated fatty acids (i.e., caprylic and capric acids) are the predominant medium chain fatty acid present in these fats, with the $C_6$ saturated fatty acid (i.e., caproic acid) being a very minor component. Typically, these reduced calorie fats contain no more than about 10%, and preferably no more than about 5% $C_6$ saturated fatty acid.

The other important aspect of the fatty acid compositions of these reduced calorie fats is the total amount of $C_{20}$ to $C_{24}$ saturated fatty acids. These very long chain fatty acids, when hydrolyzed from the respective triglyceride, are solid at body temperature, i.e. 98.6° F (37° C). Accordingly, these hydrolyzed very long chain fatty acids are much more poorly absorbed compared to the medium chain saturated fatty acids and other long chain saturated and unsaturated fatty acids.

The fatty acid composition of these reduced calorie fats can comprise from about 10 to about 70%, preferably from about 40 to about 60%, and most preferably from about 40 to about 50% $C_{20}$ to $C_{24}$ saturated fatty acids. Due to the sources of very long chain fatty acids typically used to synthesize these reduced calorie fats, behenic acid ($C_{22}$) is the predominant one present in these fats, with the $C_{20}$ and $C_{24}$ saturated fatty acids (i.e., arachidic and tetracosanoic acid) being very minor components. Typically, these reduced calorie fats comprise no more than about 10% $C_{20}$ saturated fatty acid and no more than about 2.5% $C_{24}$ saturated fatty acid, and preferably no more than about 6% $C_{20}$ saturated fatty acid and no more than about 1.5% $C_{24}$ saturated fatty acid.

These reduced calorie fats can also contain minor amounts of other fatty acids. For example, small amounts of $C_{12}$ to $C_{18}$ saturated fatty acids (e.g., lauric, myristic, palmitic and stearic acids), as well as $C_{18}$ unsaturated fatty acids (e.g., oleic, linoleic and linolenic acids), can be present in the reduced calorie fats, typically due to the sources of fatty acids used in synthesis. These other fatty acids can affect the calorie-reduction and cholesterol-lowering benefits, as well as the physical properties, of these reduced calorie fats. Accordingly, these reduced calorie fats usually comprise no more than about 9%, preferably no more than about 5%, and most preferably no more than about 3% of these other fatty acids.

These reduced calorie fats can be synthesized by a wide variety of techniques such as:

(a) random rearrangement of very long chain fatty acid triglycerides (e.g., tribehenin) and medium chain fatty acid triglycerides having $C_6$ to $C_{10}$ saturated fatty acids;

(b) esterification of glycerol with a blend of the corresponding fatty acids;

(c) transesterification of a blend of medium chain saturated fatty acid methyl esters and very long chain fatty acid methyl esters with glycerol; and

(d) transesterification of very long chain fatty acid glycerol esters (e.g., glyceryl behenate) with medium chain triglycerides.

Random rearrangement of triglycerides is well known in the art, as is the esterification of glycerol with fatty acids. For discussions on these subjects, see Hamilton et al., Fats and Oils: Chemistry and Technology , pp. 93-96, Applied Science Publishers Ltd., London (1980), and Swern, Bailey's Industrial Oil and Fat Products , 3d Ed., pp. 941-943 and 958,965 (1964), which are incorporated by reference. Transesterification is also discussed generally in Bailey's at pp. 958-963.

Fatty acids per se or naturally occurring fats and oils can serve as sources of fatty acids for preparing the reduced calorie fats. For example, hydrogenated high erucic acid rapeseed oil is a good source of behenic acid. Medium chain saturated fatty acids can be obtained from coconut, palm kernel, or babassu oils. They can also be obtained from commercial medium chain triglycerides, such as the Captex 300 Series brands sold by Capital City Products, of Columbus, Ohio.

Tribehenin, useful for making these reduced calorie fats, can be prepared from behenic acid or from fractionated methyl behenate by esterification of the acid, or by transesterification of methyl behenate with glycerol. More importantly, blends of behenic acid and medium chain saturated fatty acids can be esterified with glycerol. Similarly, methyl ester blends can also be interesterified with glycerol.

The crude triglyceride mixture resulting from synthesis is typically modified by additional fractionation to provide higher levels of mono-long chain MLM and MML triglycerides in the reduced calorie fats. Solvent and non-solvent crystal fractionation or fractional distillation methods (e.g. molecular distillation as described below) can be used. Standard fractionation methods are discussed in Applewhite, Bailey's Industrial Oil and Fat Products , Vol. 3, 4th Ed. (1985), pp. 1-39, John Wiley & Sons, New York, which is incorporated by reference. Molecular distillation can separate MML/MLM from LLM/LML triglycerides, and can shift the carbon number concentration, but it cannot fractionate triglycerides having the same carbon number. Non-solvent or solvent crystal fractionation can also fractionate MLM/MML triglycerides from the higher melting LLM/LML triglycerides. The molecular distillation or crystal fractionation of the crude triglyceride mixture is usually repeated several times to increase the level of desired MLM/MML triglycerides in these reduced calorie fats.

Fractional distillation of the crude triglyceride mixture is not limited to molecular distillation, but can also include conventional distillation (continuous or batch). After synthesis of the crude triglyceride mixture, it is common to use a conventional batch distillation technique to remove most of the excess medium chain triglycerides, and then continue with molecular distillation. The vacuum requirements are not as strict, and the temperature used can be higher in conventional distillation versus molecular distillation. The conventional distillation temperature is generally between 405° F (207° C) and 515° F (268.3° C). The absolute pressure is less than 8 mm Hg, more preferably less than 2 mm Hg. The distillation is aided by sparging with steam, nitrogen or other inert gas (e.g., $CO_2$). The distillation is carried out to remove part of the excess medium chain triglycerides, most of the excess medium chain triglycerides, or to distill also the mono-long chain (MLM and MML) components.

Crystal fractionation of the distilled triglyceride mixture can be carried out with and without solvents, with and without agitation. The crystal fractionation can be repeated several times. Crystal fractionation is particularly effective to remove high melters. Fractionation of the distilled triglyceride mixture without solvents can be used to remove LLM and LML components (predominantly carbon numbers $C_{52}$ and $C_{54}$), which in turn alters the melting profile of these reduced calorie fats.

Another preferred source of triglycerides having $C_{20}$ to $C_{24}$ saturated fatty acids are certain "hardstock fats" characterized by substantial levels of USS and SUS triglycerides, wherein U is selected from $C_{18}$ to $C_{24}$ unsaturated fatty acids and mixtures thereof, one S is selected from $C_{20}$ to $C_{24}$ saturated fatty acids and mixtures thereof, and the other S is selected from $C_{18}$ to $C_{24}$ saturated fatty acids and mixtures thereof. See U.S. applications entitled "Functional Hardstock Fat Composition" to David K. Yang, Serial No. 213,959 (P&G Case 3854), filed July 1, 1988, herein incorporated by reference, which discloses hardstock fats useful as sources of very long chain fatty acid triglycerides for the present invention, and especially Examples I and II for methods for making same.

These hardstock fats comprise at least about 8% combined USS and SUS triglycerides, preferably at least about 10% of such combined triglycerides, and most preferably 100% of such combined triglycerides. Typical commercial hardstock fats comprise from about 14 to about 35% of such combined triglycerides. In addition to the USS and SUS triglycerides, these hardstock fats often contain mixtures of other triglycerides having various configurations of unsaturated and saturated $C_{18}$ to $C_{24}$ fatty acids. These configurations include UUS, SSS, and UUU type triglycerides.

These hardstock fats can be prepared by a wide variety of techniques such as:

(a) random rearrangement of triglycerides (fats/oils);

(b) esterification of glycerol with a blend of the corresponding fatty acids; and

(c) transesterification of a blend of the corresponding fatty acid methyl esters with glycerol.

These hardstock fats are generally made by blending and randomizing various edible fats/oils, although other methods known to the art for making hardstock fats can also be used. Suitable fats/oils for preparing these hardstock fats (using a rearrangement process) include completely hydrogenated high erucic acid rapeseed oil, hydrogenated marine oils (e.g. herring and menhaden oils), safflower oil, sunflower oil, soybean oil, cottonseed oil, and corn oil.

## B. Triglyceride Fats Based on Very Long Chain Fatty Acid Triglycerides

The above sources of very long chain fatty acid triglycerides are used to prepare the triglyceride fats of the present invention. These triglyceride fats can be based solely on sources of these very long chain fatty acid triglycerides, or by blending sources of these very long chain fatty acid triglycerides with other triglycerides derived from other triglyceride fat sources.

The triglyceride fats of the present invention have fatty acid compositions comprising from about 5 to about 70% $C_{20}$ to $C_{24}$ saturated fatty acids. Preferred triglyceride fats have fatty acid compositions comprising from about 10 to about 50% $C_{20}$ to $C_{24}$ saturated fatty acids, and most preferably from about 20 to about 40% $C_{20}$ to $C_{24}$ saturated fatty acids.

The triglyceride fats of the present invention are also useful in the triglyceride fat component of fat-containing food compositions. These triglyceride fat components can comprise the triglyceride fats of the present invention, in whole or in part, e.g. the triglyceride fats of the present invention can comprise from about 30 to 100% of such fat components. These triglyceride fat components have a fatty acid composition comprising from about 10 to about 50% $C_{20}$ to $C_{24}$ saturated fatty acids. Preferably, these triglyceride fat components have a fatty acid composition comprising from about 20 to about 40% $C_{20}$ to $C_{24}$ saturated fatty acids.

The triglyceride fats that are useful in these triglyceride components are based upon the above previously described reduced calorie fats, hardstock fats, or blends thereof. One such preferred triglyceride fat comprises from from about 10 to about 90%, preferably from about 30 to about 60%, and most preferably from about 30 to about 50% combined MLM, MML, LLM and LML triglycerides (as previously defined), with from about 10 to about 90%, preferably from about 40 to about 70, and most preferably from about 50 to about 70%, combined USS and SUS triglycerides (as previously defined).

The triglyceride fats of the present invention that are useful in these triglyceride fat components can be blended with other triglyceride fat sources to form the triglyceride fat component. These other triglyceride fat sources include animal, vegetable or marine fat or oil sources, particularly vegetable oils such as cottonseed oil, soybean oil, sunflower oil, corn oil, peanut oil, safflower oil, rapeseed oil, canola oil, and the like. Triglyceride oils most preferably used are soybean oil, safflower oil, sunflower oil, canola oil, and blends thereof.

## C. Pharmaceutical and Food Uses of Triglyceride Fats

The triglyceride fats of the present invention can be used as a pharmaceutical agent to inhibit the absorption of cholesterol. In particular, pharmaceutical compositions in effective unit dosage amounts of from about 1 to about 20 grams, and preferably from about 2 to about 10 grams, of the triglyceride fats of the present invention can be formulated to inhibit the absorption of cholesterol. In addition, the triglyceride fats of the present invention are useful in methods for treating hypercholesterolemia. Such methods involve systemically administering to an animal susceptible to or afflicted with hypercholesterolemia successive therapeutically doses of the triglyceride fats of the present invention as a pharmaceutical agent.

In therapeutic regimens, the dosage of the triglyceride fats of the present invention can vary with the severity of the hypercholesterolemic conditions and the duration of treatment. Individual dosages can range from about 25 to about 150 mg. per kg. (Unless otherwise specified, the unit "mg. per kg." is used herein to refer to milligrams of triglyceride fat per kilogram of body weight.) Preferably, the triglyceride fats of the present invention are administered in amounts of from about 25 to about 75 mg. per kg. per dosage, with up to 4 dosages, preferably 3 dosages, being given daily, most preferably at meal times. Dosages of less than about 20 mg. per kg. do not materially inhibit the absorption of cholesterol in most patients. The dosages can sbe administered orally in any suitable unit dosage form such as chewable tablets, wafers or emulsion drinks. See U.S. Patent 4,368,213, to Hollenbach et al, issued January 11, 1988 (herein incorporated by reference) for the preparation of emulsion drinks. These dosages can also be administered as part of a controlled dietary regimen, e.g. less than 7% calories as saturated fat, no more than 10% calories as polyunsaturated fat, 10-15% calories as monounsaturated fat, less than 200 mg./day cholesterol and total calories in the diet to achieve desired body weight.

The pharmaceutical compositions of the present invention can comprise the triglyceride fat as the pharmaceutical agent, alone, or in combination with any desired, non-interfering pharmaceutical carrier. As used herein, the term "pharmaceutical carrier" means a solid or liquid filler, diluent or encapsulating substance. Some examples of substances which can serve as pharmaceutical carriers are sugar such as lactose, glucose and sucrose; starches such as cornstarch and potato starch; cellulose and its derivatives, such as sodium carboxymethylcellulose, ethylcellulose, cellulose acetate; powdered tragacanth; malt; gelatin; talc; polyols such as propylene glycol, glycerine, sorbitol, mannitol and polyethylene glycol; agar; alginic acid; pyrogen-free water; isotonic saline; ethyl alcohol and phosphate buffer solutions, as well as other non-toxic compatible substances used in pharmaceutical formulations. Wetting agents and lubricants such as sodium lauryl sulfate, as well as coloring agents, flavoring agents and preservatives, can also be present in such pharmaceutical compositions, according to the desires of the formulator.

The pharmaceutical carriers of the foregoing type can optionally be employed in conjunction with the triglyceride fats of the present invention to provide a practical size dosage relationship, composition forms which can be easily ingested, and means for providing accurate unit dosages in a convenient form. The pharmaceutical carrier can comprise from about 10 to about 90% of the total pharmaceutical composition; and typically comprises from about 10 to about 50% of the total composition.

The triglyceride fats previously described are also useful, in whole or in part, in the triglyceride fat component of fat-containing food compositions. These fat-containing food compositions can comprise from about 10 to 100% of the triglyceride fat component of the present invention. Within this range, the level of fat component present in these food compositions can vary greatly depending upon the product form involved and the particular cholesterol-lowering properties desired. Typically, these fat-containing food compositions comprise from about 20 to 100% of the triglyceride fat component of the present invention.

The triglyceride fat components of the present invention are typically used as replacements for the normal triglyceride fat present in fat-containing food compositions to provide the desired cholesterol-lowering benefits. As such, these triglyceride fat components can be used as a cocoa butter substitute. As used herein, the term "cocoa butter substitute" refers to a fat which is used to totally or partially replace cocoa butter in confectionery fat products such as chocolate. More typically, the triglyceride fat components of the present invention are used as other than cocoa butter substitutes. Some representative fat-containing food compositions which can comprise the triglyceride fat components of the present invention include shortening and oil products such as plastic shortenings, emulsified spreads, margarines, butter blends, lards, salad oils, cooking oils, frying oils, popcorn oils, salad dressings, whipped toppings, peanut butter, and frostings; baking mixes, other prepared mixes and baked goods such as cakes, brownies, muffins, bar cookies, wafers, biscuits, pastries, pies, piecrusts, and cookies such as sandwich cookies and chocolate chip cookies, filled baked goods containing fruit, creme or other fillings, breads, rolls, crackers, pretzels, pancakes, waffles, ice cream cones and cups, yeast-raised baked goods, pizzas and pizza crust, baked farinaceous snack foods and other baked salted snack products; salted and fried snacks such as potato chips, potato sticks, corn chips, tortilla chips, taco chips and the like; frozen desserts such as ice cream; processed meat products; and processed vegetable products.

The fat-containing food compositions of the present invention can be used as part of a dietary method or regimen for lowering blood cholesterol levels when ingested in a cholesterol-lowering amount. What will constitute a "cholesterol-lowering amount" of such fat-containing food compositions will vary greatly depending upon the diet of the particular individual, the susceptibility of the particular individual to the adverse effects of cholesterol absorption, and the particular cholesterol-lowering benefits desired. For example, those individuals who are prone to being hypercholesterolemic may need to ingest greater quantities of the fat-containing food compositions of the present invention in order to achieve the desired cholesterol-lowering benefits. In any event, the attending physician can, if so desired, measure the particular individual's blood cholesterol level both before and after ingestion of the fat-containing food compositions of the present invention. Based on such data, the appropriate amount of fat-containing food composition for the individual's particular diet can then be determined. Generally, the mg. per kg. dosages previously described for pharmaceutical compositions can be used as a starting point for determining the amount of fat-containing food composition that should be ingested in order to obtain the desired cholesterol-lowering benefits.

The triglyceride fats of the present invention can be fortified with vitamins and minerals, particularly the fat-soluble vitamins. U.S. Patent 4,034,083 of Mattson (incorporated by reference herein) discloses polyol fatty acid polyesters fortified with fat-soluble vitamins. The fat-soluble vitamins include vitamin A, vitamin D, vitamin E, and vitamin K. Vitamin A is a fat-soluble alcohol of the formula $C_{20}H_{29}OH$. Natural vitamin A is usually found esterified with a fatty acid; metabolically active forms of vitamin A also include the corresponding aldehyde and acid. Vitamin D is a fat-soluble vitamin well known for use in the treatment and prevention of rickets and other skeletal disorders. Vitamin D comprises sterols, and there are at least 11 sterols with vitamin D-type activity. Vitamin E (tocopherol) is a third fat-soluble vitamin which can be used in the present invention. Four different tocopherols have been identified (alpha, beta, gamma and delta), all of which are oily, yellow liquids, insoluble in water but soluble in fats and oils. Vitamin K exists in at least three forms, all belonging to the group of chemical compounds known as quinones. The naturally occurring fat-soluble vitamins are $K_1$ (phylloquinone), and $K_2$ (menaquinone), while vitamin $K_3$ (menadione) is synthetically derived. The amount of the fat-soluble vitamins employed herein to fortify the present triglyceride fats can vary. If desired, these triglyceride fats can be fortified with a recommended daily allowance (RDA), or increment or multiple of an RDA, of any of the fat-soluble vitamins or combinations thereof.

Vitamins that are nonsoluble in fat can similarly be included in the triglyceride fats of the present invention. Among these vitamins are the vitamin B complex vitamins, vitamin C, vitamin G, vitamin H, and vitamin P. The minerals include the wide variety of minerals known to be useful in the diet, such as calcium, magnesium, and zinc. Any combination of vitamins and minerals can be used in the present triglyceride fats.

The triglyceride fats of the present invention can be used in combination with various classes of food ingredients. For example, an extra calorie reduction benefit can be achieved when noncaloric or reduced calorie sweeteners are used, alone or in combination with bulking agents. Noncaloric or reduced calorie sweeteners include, but are not limited to, aspartame; saccharin; alitame, thaumatin; dihydrochalcones; cyclamates; steviosides; glycyrrhizins, synthetic alkoxy aromatics, such as Dulcin and P-4000; sucrolose; suosan; miraculin; monellin; sorbitol, xylitol; talin; cyclohexyl-sulfamates; substituted imidazolines; synthetic sulfamic acids such as acesulfame, acesulfam-K and n-substituted sulfamic acids; oximes such as perilartine; rebaudioside-A; peptides such as aspartyl malonates and succanilic acids; dipeptides; amino acid based sweeteners such as gem-diaminoalkanes, meta-aminobenzoic acid, L-aminodicarboxylic acid alkanes, and amides of certain alpha-aminodicarboxylic acids and gem-diamines; and 3-hydroxy-4-alkyloxyphenyl aliphatic carboxylates or heterocyclic aromatic carboxylates.

The triglyceride fats of the present invention can be used in combination with other noncaloric or reduced calorie fats, such as branched chain fatty acid triglycerides, triglycerol ethers, polycarboxylic acid esters, sucrose polyethers, neopentyl alcohol esters, silicone oils/siloxanes, and dicarboxylic acid esters. Other partial fat replacements useful in combination with these triglyceride fats are medium chain triglycerides, highly esterified polyglycerol esters, acetin fats, plant sterol esters, polyoxyethylene esters, jojoba esters, mono/diglycerides of fatty acids, mono/diglycerides of short-chain dibasic acids, and certain edible, substantially nonabsorbable, substantially nondigestible polyol fatty acid polyester having at least 4 fatty acid ester groups, wherein the polyol is selected from sugars and sugar alcohols containing from 4 to 8 hydroxy groups and wherein each fatty acid group has from 2 to 24 carbon atoms. See, for example, U.S. Patent 3,600,186 to Mattson et al, issued August 17, 1971 (herein incorporated by reference) for a representative disclosure of such polyol polyesters.

Bulking or bodying agents can also be used in combination with the triglyceride fats of the present invention in many fat-containing food compositions. The bulking agents can be nondigestible carbohydrates, for example, polydextrose and cellulose or cellulose derivatives, such as carboxymethylcellulose, carboxyethylcellulose, hydroxypropylcellulose, methylcellulose and microcrystalline cellulose. Other suitable bulking agents include gums (hydrocolloids), starches, dextrins, fermented whey, tofu, maltodextrins, polyols, including sugar alcohols, e.g. sorbitol and mannitol, and carbohydrates, e.g. lactose.

Similarly, fat-containing food compositions can be made that combine the triglyceride fats of the present invention with dietary fibers to achieve the combined benefits of each. By "dietary fiber" is meant complex carbohydrates resistant to digestion by mammalian enzymes, such as the carbohydrates found in plant cell walls and seaweed, and those produced by microbial fermentation. Examples of these complex carbohydrates are brans, celluloses, hemicelluloses, pectins, gums and mucilages, seaweed extract, and biosynthetic gums. Sources of the cellulosic fiber include vegetables, fruits, seeds, cereals, and man-made fibers (for example, by bacterial synthesis). Commercial fibers such as purified plant cellulose, or cellulose flour, can also be used. Naturally occurring fibers include fiber from whole citrus peel, citrus albedo, sugar beets, citrus pulp and vesicle solids, apples, apricots, and watermelon rinds.

These dietary fibers may be in a crude or purified form. The dietary fiber used may be of a single type (e.g. cellulose), a composite dietary fiber (e.g. citrus albedo fiber containing cellulose and pectin), or some combination of fibers (e.g. cellulose and a gum). The fibers can be processed by methods known to the art.

The triglyceride fats of the present invention and food compositions containing same can also contain minor amounts of optional flavorings, emulsifiers, anti-spattering agents, anti-sticking agents, anti-oxidants, or the like.

Of course, judgment should be exercised to make use of appropriate combinations of these triglyceride fats with other food ingredients. For example, a combination of sweetener and fat would not be used where the specific benefits of the two are not desired. The fat ingredient combinations are used where appropriate, and in the proper amounts.

Analytical Methods for Reduced Calorie Fats

A. Carbon Number Profile (CNP)

1. CNP/HPLC Method

The carbon number profile of the triglycerides comprising the reduced calorie fats of the present invention can be measured by high performance liquid chromatography (HPLC). The method also measures the percentages of medium chain triglycerides, mono-long chain and di-long chain triglycerides. A triglyceride sample to be analyzed is injected on a reverse phase liquid chromatograph (LC) equipped with a mass (evaporative light scattering) detector. A linear gradient of increasing methylene chloride in acetonitrile is used to separate all of the triglycerides based on fatty acid chain length. Retention time increases with increasing fatty acid chain length. Thus, medium chain triglycerides are eluted first, followed by mono-long chain and then di-long chain triglycerides.

**Apparatus**

| Dispensers | 1 mL, American Scientific #P4952-1, or equivalent, American Scientific Products, 1430 Waukegan Rd., McGaw Park, IL 60085 |
| --- | --- |

| | |
|---|---|
| Pasteur pipets, glass | Fisher #13-678-7A, or equivalent, Fisher Scientific Co., 203 Fisher Bldg., Pittsburgh, PA 15219 |
| Vials, glass | 2 dram with foil-lined cap |
| Autosampler vials | 2 mL, Fisher #03-340-SG, Fisher Scientific Co. |
| Vial caps | PTFE Rubber, Fisher #03-340-13C, Fisher Scientific Co. |
| LC columns | 2 Beckman Ultrasphere ODS, 5 um, 0.46 cm i.d. x 25 cm, Beckman Instruments, Inc., 2500-T Harbor Blvd., Fullerton, CA 92634 |
| LC system | Hewlett-Packard 1090L with Ternary DR5 pump, variable volume injector, autosampler, heated column compartment and column switching valve, Hewlett-Packard Co., Scientific Instruments Div., 1601-T California Ave., Palo Alto, CA 94304 |
| Mass detector | Applied Chromatography Systems #750/14, Varex Corp., 12221 Parklane Dr., Rockville, MD 20852 |
| Recorder | Kipp & Zonen #BD40, or equivalent, Kipp & Zonen, Div. of Enraf-Nonius, 390-T Central Ave., Bohemia, NY 11716 |
| Laboratory Automation System (LAS) | Hewlett-Packard 3357, or equivalent, Hewlett-Packard Co., Scientific Instruments Div. |
| Filters | Gelman #4451, 0.2 um, or equivalent, Gelman Instrument Co., 605-T S. Wagner Rd., Ann Arbor, MI 48106 |
| Solvent Clarification kit | Waters #85124, Waters Instruments, Inc., 2411-T 7th St. N.W., Rochester, MN 55901 |

| Syringe | 5 ml, disposable, Fisher #14-823-200, or equivalent, Fisher Scientific Co. |

### Reagents

| Methylene chloride | Burdick and Jackson, UV Grade, American Scientific #300-4L, American Scientific Products |
| Acetonitrile | Burdick and Jackson, UV Grade, American Scientific #015-4L, American Scientific Products |

### Sample Preparation

1. Weigh 0.1 g of the melted sample into a 2 dram vial.
2. Dispense 1 mL of methylene chloride into vial and mix thoroughly.
3. Filter the sample solution through a 0.2 um filter into an autosampler vial.

### LAS Method and Sequence Preparation

1. Set up the integration method, referring to the HP-3357 Quick Reference Guide for instructions. The calibration table is shown in Table 2.
2. Set up a LAS sample sequence for the appropriate number of samples. Refer to the Reference Guide as necessary.

TABLE 2

| | | Calibration Table | | |
|---|---|---|---|---|
| | Time | Factor | Amount | Peak Name |
| 1. | 3.48 | 1.000000 | 1.000000 | C22 |
| 2. | 3.80 | 1.000000 | 1.000000 | C24 |
| 3. | 4.18 | 1.000000 | 1.000000 | C26 |
| 4. | 4.30 | 1.000000 | 1.000000 | C28 |
| 5. | 4.65 | 1.000000 | 1.000000 | C30 |
| 6. | 5.32 | 1.000000 | 1.000000 | C32 |
| 7. | 6.01 | 1.000000 | 1.000000 | C34 |
| 8. | 6.80 | 1.000000 | 1.000000 | C36 |
| 9. | 7.87 | 1.000000 | 1.000000 | C38 |
| 10. | 8.98 | 1.000000 | 1.000000 | C40 |
| 11. | 10.31 | 1.000000 | 1.000000 | C42 |
| 12. | 11.88 | 1.000000 | 1.000000 | C44 |
| 13. | 13.49 | 1.000000 | 1.000000 | C46 |
| 14. | 15.35 | 1.000000 | 1.000000 | C48 |
| 15. | 17.28 | 1.000000 | 1.000000 | C50 |
| 16. | 19.49 | 1.000000 | 1.000000 | C52 |
| 17. | 21.60 | 1.000000 | 1.000000 | C54 |
| 18. | 23.87 | 1.000000 | 1.000000 | C56 |
| 19. | 26.18 | 1.000000 | 1.000000 | C58 |
| 20. | 28.50 | 1.000000 | 1.000000 | C60 |
| 21. | 30.77 | 1.000000 | 1.000000 | C62 |
| 22. | 33.03 | 1.000000 | 1.000000 | C64 |
| 23. | 35.24 | 1.000000 | 1.000000 | C66 |

LC Operation

A. Start-up

1. Turn on power for the HP1090.
2. Filter all solvents with filtration apparatus.
3. Fill reservoirs with filtered solvent; reservoir A contains acetonitrile and reservoir B contains methylene chloride. Open helium toggle valve on back of LC and degas solvents for at least 5-10 minutes. Close helium toggle valve.
4. Set the mass detector to the following settings:

Attenutation: 2
Photomultiplier: 2
Time Constant: 5
Evaporator Setting: 50
Nitrogen: 12 psi

5. Set up the mobile phase gradient method in Table 3 on the HP1090 as necessary. Refer to HP1090 Operator's Handbook for programming directions. Once the method is programmed, it will remain in the memory until it is erased, even with power off or instrument unplugged.

## Table 3
## Mobile Phase Gradient Program

```
METHOD 1
TMCT
SDS CONFIG A = 1 B = 1 C = 0
FLOW = 2
%B=35 C = 0
OVEN = 40 INJ VOL = 10 SLOWDOWN = 5
MAX PRESS = 300
MIN PRESS = 0
STOP TIME = 40.1
POST TIME = 5
COLUMN SWITCH = 0
E = 0 0 0 0
AT  0  E4 = 1
AT  0  %B = 35  %C = 0
AT  .1  E4 = 0
AT 40  %B = 55  %C = 0
```

B. Autosampler Operation

1. Place the filled autosampler vials in autosampler holders starting with space "0". Autosampler starts numbering with "0" and the LAS starts numbering with "1", thus the sequence numbers are shifted by one.
2. Program and start the autosampler for number of injections, refer to handbook.

Reference Standards

A reference standard is used to insure proper LC/detector operation and to verify the identification of the triglyceride peaks. Typically, a well-characterized material is used. When such material is not available, a commercial material such as Nu Chek Prep 50A and 51A can be substituted (Nu Chek Prep, Inc., P.O. Box 172, Elysian, MN 56028). The reference standard is analyzed each day prior to sample analyses.

Results

1. As each sample is analyzed, the LAS will generate a report according to the instructions of the integration method (Table 2). The report lists peak number, retention time, and area percent for a given carbon number of the triglyceride sample.
2. Since retention times of peaks will shift as a function of column usage, verify the proper identification of the reference standards peaks. If peaks are mislabelled, modify the retention time table of the integration method and reanalyze the sequence to generate the new reports.
3. A chromatogram is often helpful to understand the data. Use CPLOT to generate a chromatogram.

2. CNP/GC Method

The carbon number profile (CNP) of the triglycerides comprising the reduced calorie fat of the present invention can also be determined by programmed temperature-gas chromatography (GC) using a short fused silica column coated with methyl silicone for analysis and characterization of the composition by molecular weight. The glycerides are separated according to their respective carbon numbers, wherein the carbon number defines the total number of carbon atoms on the combined fatty acid residues. The carbon atoms on the glycerol molecule are not counted. Glycerides with the same carbon number will elute as the same peak. For example, a triglyceride composed of three $C_{16}$ (palmitic) fatty acid residues will co-elute with triglycerides made up of one $C_{14}$ (myristic), one $C_{16}$ and one $C_{18}$ (stearic) fatty acid residue or with a triglyceride composed of two $C_{14}$ fatty acid residues and one $C_{20}$ (arachidic) fatty acid residue.

Preparation of the fat sample for analysis is as follows: 1.0 ml. of a tricaprin internal standard solution (2 microg./ml.) is pipetted into a vial. The methylene chloride solvent in the standard solution is evaporated using a steam bath under a nitrogen stream. Two drops of the fat sample (20 to 40 microg.) are pipetted into a vial. If the fat sample is solid, it is melted on a steam bath and stirred well to insure a representative sample. 1.0 ml. of bis (trimethylsilytrifluoroacetamide) (BSTFA) is pipetted into the vial which is then capped. The contents of the vial are shaken vigorously and then placed in a beating block (temperature of $100°$ C) for about 5 minutes.

For determining the CNP-GC of the prepared fat samples, a Hewlett-Packard 5880A series gas chromatograph equipped with temperature programming and a hydrogen flame ionization detector is used together with a Hewlett-Packard 3351B data system. A 2 m. long, 0.22 mm. diameter fused silica capillary column coated with a thin layer of methyl silicone (Chrompak CP-SIL 5) is also used. The column is heated in an oven where temperature can be controlled and increased according to a specified pattern by the temperature programmer. The hydrogen flame ionization detector is attached to the outlet port of the column. The signal generated by the detector is amplified by an electrometer into a working input signal for the data system and recorder. The recorder prints out the gas chromatograph curve and the data system electronically integrates the area under the curve. The following instrument conditions are used with the gas chromatograph:

| | |
|---|---|
| Septum purge | 1 ml./min. |
| Inlet pressure | 5 lbs./in.$^2$ |
| Vent flow | 75 ml./min |
| Makeup carrier | 30 ml./min |
| Hydrogen | 30 ml./min |
| Air | 400 ml./min |

1.0 microl. of the prepared fat sample is taken by a gas-tight syringe and injected into the sample port of the gas chromatograph. The components in the sample port are warmed up to a temperature of $365°$ C and swept by a helium carrier gas to push the components into the column. The column temperature is initially set at $175°$ C and held at this temperature for 0.5 min. The column is then heated up to a final temperature of $355°$ C at a rate of $25°$ C/min. The column is maintained at the final temperature of $355°$ C for an additional 2 min.

The chromatographic peaks generated are then identified and the peak areas measured. Peak identification is accomplished by comparison to known pure glycerides previously programmed into the data system. The peak area as determined by the data system is used to calculate the percentage of glycerides having a particular Carbon Number ($C_N$) according to the following equation:

% $C_N$ = (Area of $C_N$/S) x 100

wherein S = sum of Area of $C_N$ for all peaks generated.

The Area of $C_N$ is based upon the actual response generated by the chromatograph multiplied by a response factor for glycerides of the particular Carbon Number. These response factors are determined by comparing the actual responses of a mixture of pure glycerides of various Carbon Numbers to the known amounts of each glyceride in the mixture. A glyceride generating an actual response greater than its actual amount has a response factor less than 1.0; likewise, a glyceride generating a response less than that of its actual amount has a response factor of greater than 1.0. The mixture of glycerides used (in a methylene chloride solution) is as follows:

16

| Component | Carbon No. | Amount (mg.ml.) |
|---|---|---|
| Palmitic acid | 16 | 0.5 |
| Monopalmitin | 16 | 0.5 |
| Monostearin | 18 | 0.5 |
| Dipalmitin | 32 | 0.5 |
| Palmitostearin | 34 | 0.5 |
| Distearin | 36 | 0.5 |
| Tripalmitin | 48 | 1.5 |
| Dipalmitostearin | 50 | 1.5 |
| Distearopalmitin | 52 | 1.5 |
| Tristearin | 54 | 1.5 |

B. Fatty Acid Composition

Principle

The fatty acid composition of the triglycerides comprising the reduced calorie fat of the present invention is measured by gas chromatography. First, fatty acid ethyl esters of the triglycerides are prepared by any standard method (e.g., by transesterification using sodium ethoxide), and then separated on a capillary column which is coated with DB-WAX stationary phase. The fatty acid ethyl esters are separated by chain length and degree of unsaturation. A split injection is made with flame ionization detection. Quantitation is performed by use of a double internal standard method. This method can separate fatty acid ethyl esters from C6 to C24.

| Equipment | |
|---|---|
| Gas Chromatograph | Hewlett-Packard 5890, or equivalent, equipped with a split injector and flame ionization detector, Hewlett-Packard Co., Scientific Instruments Div., 1601-T California Ave., Palo Alto, CA 94304 |
| Autosampler Injector column | Hewlett-Packard 7673A, or equivalent |
| Column | 15 m x 0.25 mm I.D., fused silica capillary column coated with DB-WAX (0.25 micron film thickness), Hewlett-Packard Co., Scientific Instruments Div. |
| Data System | Hewlett-Packard 3350, 3000-T Hanover St., Palo Alto, CA 94304 |
| Recorder | Kipp & Zonen, BD40, Kipp & Zonen |

| Reagent | |
|---|---|
| Hexane | Burdick & Jackson, or equivalent, American Scientific Products |

Reference Standards

Two reference standards are used each day of operation to verify proper operation of this method. 1) A reference mixture of fatty acid methyl esters (FAME) is used to check the operation of the instrument. This reference mixture has the following fatty acid composition: 1% $C_{14:0}$, 4% $C_{16:0}$, 3% $C_{18:0}$, 45% $C_{18:1}$, 15% $C_{18:2}$, 3% $C_{18:3}$, 3% $C_{20:0}$, 3% $C_{22:0}$, 20% $C_{22:1}$, and 3% $C_{24:0}$. 2) A reference standard of a commercial shortening is used to check the operaton of the total system -- ethylation and gas chromatographic analysis. The shortening referenoe standard has the following fatty acid composition: 0.5% $C_{14:0}$, 21.4% $C_{16:0}$, 9.2% $C_{18:0}$, 40.3% $C_{18:1}$, 23.0% $C_{18:2}$, 2.2% $C_{18:3}$, 0.4% $C_{10:0}$, 1.3% $C_{20:1}$, and 0.3% $C_{22:0}$.

The reference mixture of FAME should be diluted with hexane and then injected into the instrument. A new vial of FAME reference mixture should be opened every day since the highly unsaturated components,

$C_{18:2}$ and $C_{18:3}$, oxidize easily. The shortening reference standard should be ethylated with the samples prior to their analysis by capillary gas chromatography. The results from the reference standards should be compared with the known values and a judgment made concerning the completed analysis. If the results of the reference standards are equal to or within ± standard deviations of the known values, then the equipment, reagents and operations are performing satusfactorily.

Operation

A. Instrumental Set-up

1. Install the column in the gas chromatograph, and set up the instrumental conditions as in Table 4.
2. Set up the data system with the appropriate method to acquire and analyze the data. The retention times may have to be adjusted in the method due to instrument variations. Consult the data system reference manual on how to do this -- HP3350 User's Reference Manual. Unity response factors are used for each component.
3. Obtain the shortening reference standard for analysis with the samples and ethylate it with the samples.

Table 4

| INSTRUMENTAL CONDITIONS | |
|---|---|
| Instrument | Hewlett-Packard 5890 |
| Column | 15 m x 0.25 mm I.D., coated with DB-WAX, 0.25 u film thickness |
| Column head pressure | 12.5 psi |
| Carrier gas | Helium |
| Injector "A" temperature | 210° C (410° F) |
| Split vent flow | 100 mL/min |
| Septum purge | 1.5 mL/min |
| Oven temperature profile: | |
| Initial temperature | 110° C (230° F) |
| Initial time | 1 min |
| Rate 1 | 15° C/min |
| Final temp 1 | 170° C (338° F) |
| Final time 1 | 0 min |
| Rate 2 | 6° C/min |
| Final temp 2 | 200° C (392° F) |
| Final time 2 | 0 min |
| Rate 3 | 10° C/min |
| Final temp 3 | 220° C (428° F) |
| Final time 3 | 8 min |
| Detector | FID |
| Detector temp | 230° C (446° F) |
| Make-up gas | 30 mL/min |
| Detector $H_2$ flow | 30 mL/min |
| Detector air flow | 300 mL/min |

B. Analysis of Samples - (The samples are analyzed with a double internal standard.)

1. Dilute the reference mixture of FAME with hexane. The methyl esters should be approximately 2% in hexane. Inject one microliter of this solution via the autosampler. The results must meet the criteria in the Reference Standards section.

2. Prepare the triglyceride samples to be analyzed by adding two different internal standards, $C_9$ and $C_{21}$ triglycerides. ($C_9$ and $C_{21}$ triglycerides are commercial standards consisting of 100% 9-carbon and 21-carbon triglycerides, respectively.) The internal standards are added to the samples at about 10% by weight of the sample. The samples (including the internal standards) are then converted to ethyl esters by any standard method.

3. Set up a sequence in the LAS data system to inject the samples.

4. Activate the autosampler to inject 1.0 microl. of the samples in the sequence. The gas chromatograph will automatically begin its temperature program and the data system will collect and analyze the data for the sequence.

5. The data is analyzed with the two internal standard procedure. The absolute amount (mg of esters per gram of sample) of the $C_6$ through $C_{16}$ components is calculated from the $C_9$ internal standard. The absolute amount of the $C_{18}$, $C_{20}$, $C_{22}$ and $C_{24}$ components is calculated from the $C_{21}$ internal standard. Weight percentages of fatty acids are calculated from these amounts.

Specific Illustrations of Food and Pharmaceutical Compositions

The following are specific illustrations of food and pharmaceutical compositions according to the present invention:

A. Triglyceride Fats

1. Preparation of Reduced Calorie Fat

Compritol 888 (a mixture of approximately 25% monobehenin, 50% dibehenin and 25% tribehenin, sold by Gattefosse of 200 Sawmill River Road, Hawthorne, New York) is further esterified at 265° C with capric fatty acid until the diglyceride concentration of the mixture is reduced to less than 4%. The weight ratio of Compritol 888 to capric fatty acid at the start of esterification is approximately 70:30. The resulting esterified mixture is deodorized at 260° C for 3 hours and then combined with Captex 355 (a mixture of $C_8/C_{10}$ medium chain triglycerides, sold by Capital City Products, of Columbus, Ohio) in a weight ratio of 58:42. This mixture is randomly rearranged (randomized) at a temperature of 80° C for 20 minutes using 0.06% sodium methoxide as the catalyst, neutralized with phosphoric acid and then filtered to remove sodium phosphate. The randomized mixture (approximately 2.5% diglycerides, 38.5% medium chain (MMM) triglycerides, 43.5% mono-long chain (MLM/MML) triglycerides, 13.5% di-long chain (LLM/LML) triglycerides, and 1% tri-long chain (LLL) triglycerides), is steam stripped at a temperature of 450° to 515° F (232.2° to 268.3° C) during which a major portion of the medium chain triglycerides are distilled off. The stripped residue (2.5% diglycerides, 6% medium chain triglycerides, 67% mono-long chain triglycerides, and 24% di-long chain triglycerides) is then passed three times at gradually increasing temperatures through two 14 inch molecular stills (connected in series) to increase the level of mono-long chain triglycerides. The molecular stills are operated under the following conditions:

Bell jar pressure: 5-11 microns Hg. abs.
Rotor feed temperature: 125°-160° C
Rotor residue temperature: 180°-216° C
Initial feed pump rate: 36-40 lbs./hour
Distillation rate: 4-6 lbs./hour per unit

The distillate fractions obtained (total of 25) contain 1% medium chain triglycerides, 92% mono-long chain triglycerides, and 5-6% di-long chain triglycerides. Each of these distillate fractions are subjected to nonsolvent fractionation, first at 80° F (26.7° C) and then at 76° F (24.4° C). The liquid (olein) fractions obtained are combined to provide a reduced calorie fat having the following carbon number profile (CNP):

| CNP | % |
|---|---|
| 32 | 0.1 |
| 34 | 0.5 |
| 36 | 1.7-2.0 |
| 38 | 21.7-22.9 |
| 40 | 48.0-48.6 |
| 42 | 23.9-24.7 |
| 44 | 0.7-1.0 |
| 46 | 0.2 |
| 48 | 0.2 |
| 50 | 0.2 |
| 52 | 0.1 |

## 2. Preparation of Hardstock Fat

### a. Synthesis of diglyceride (behenoyl-1-stearoyl-glycerol)

100 g. (279.3 mmol.) of 1-monostearin is dissolved in 750 ml. of warm chloroform (Note: the chloroform is washed three times with distilled water, dried over $MgSO_4$, and filtered before use). 24.8 ml. (307.2 mmol.) of pyridine is then added to the solution. (Note: the pyridine is dried over 3A molecular sieves, manufactured by Linde, before use). 110 g. (307.2 mmol.) of behenoyl chloride is dissolved in 250 ml. of washed chloroform and added dropwise to the well-stirred solution. The reaction is gently warmed with a heating mantel and is stirred overnight (16 hrs.). Upon completion of the reaction, the solvent is removed under reduced pressure. The remaining solid/oil is transferred to a 2 liter beaker and 1 liter of acetone/ethanol 50:50 is added. The mixture is heated to boiling and the remaining solids filtered. The filtrate is then placed in a 50° C incubator overnight and filtered. The filtrate is cooled to room temperature and any solid diglyceride is removed by suction filtration.

### b. Synthesis of triglyceride (linoleoyl-behenoyl-1-stearoyl-glycerol)

63 g. (92.5 mmol.) of diglyceride from step 1 is dissolved in 1 liter of washed chloroform followed by 11.2 ml. (138.8 mmol.) of dried pyridine. After the solution is warmed slightly, 41.5 g. (138.8 mmoo.) of linoleoyl chloride is added dropwise to the well-stirred solution. The reaction flask is blanketed with nitrogen and stirred at room temperature. Thin Layer Chromatography (TLC) is used to confirm the formatin of the triglyceride (Rf = 0.72, pet ether/ethyl ether/acetic acid, 75:25:1). After 3 days, the reaction mix is placed in a 2 liter separatory funnel and washed two times with 500 ml. water. The organic layer is dried over 12-28 mesh silica gel and filtered. Excess solvent is removed under reduced pressure, which results in an oil. The oil is recrystallized in 1 liter of acetone at room temperature. The triglyceride solid is collected by suction filtration and recrystallized again in acetone. The final product is vacuum dried and stored under nitrogen at -20° C. 44 g. of linoleoyl-behenoyl-1-stearoyl-triglyceride (95% purity) is typically produced.

## B. Food Compositions

### 1. Shortening Formulation

The following formula is useful for providing a stable plastic shortening when prepared using the conventional methods:

| Canola oil (I.V. 90) | 58% |
|---|---|
| Reduced calorie fat | 19% |
| Hardstock fat | 19% |
| Mono and diglycerides (as emulsifiers) | 4% |

The ingredients are combined in a clean vessel and heated above the melting point of the reduced calorie and hardstock fats to form a melt. 15 volume percent of edible gas is injected into the melt. The melted shortening is passed through a scraped wall heat exchanger at 15° C to form a supercooled mixture. The shortening is mildly agitated while crystallization continues. Finally the shortening is tempered at 30° C for 36 hours.

2. Margarine-Like Spread

The aqueous phase of the margarine-like spread is formulated from the following ingredients:

| Ingredient | Grams |
|---|---|
| Water | 150 |
| Dstilled mono- and diglycerides | 1.5 |
| Lecithin | 1.0 |
| Natura/artificial butter flavors | 0.09 |
| Salt | 11.0 |
| Potassium sorbate | 0.12 |
| Citric acid | 0.04 |

The above aqueous phase ingredients are dissolved in the water and then heated to 130° F (54.4° C). The fat phase of the spread is formulated from the following ingredients:

| Ingredient | Grams |
|---|---|
| Reduced calrie fat | 299.1 |
| Hardstock fat | 199.4 |
| Canola oil (I.V. 90) | 332.3 |

The aqueous phase ingredients are blended into the fat phase ingredients (830.7 g.) at 130° F (54.4° C) under high shear mixing conditions using an Agi mixer equipped with a homogenizer head, a rotating bowl and Teflon scrapper blades to remove emulsified and crystallized material from the inside wall of the bowl. Chilled water is sprayed on the outside wall of the bowl to cool it. As the mass in the bowl is cooled to approximately 67° F (19.4° C), the viscosity increases to that of a typical soft margarine consistency. The emulsified/ crystallized material is filled into plastic tubs, placed in a 32° F (0° C) bath for 1 hour and then stored for 48 hours in a 40° F (4.4° C) constant temperature room to provide a soft, spreadable margarine-like product.

C. Pharmaceutical Compositions

1. Wafers

Wafers comprising a unit dosage amount of the hardstock fat (5 grams) are formulated from sucrose (9.6 grams), flour (4.8 grams), water (4.8 grams), egg white (0.25 grams) and lecithin emulsifier (0.25 grams) by conventional methods. These wafers are administered orally three times daily over a one-month period

22

in a treatment regimen to substantially inhibit cholesterol uptake and to decrease the serum level of cholesterol.

2. Emulsion Drink

A beverage concentrate is prepared, having the following composition:

| Ingredient | % w/w |
|---|---|
| Water | 26.3 |
| Potassium sorbate | 0.06 |
| Xanthan gum | 1 |
| Sucrose | 10.5 |
| Citric acid | 0.04 |
| Polyglycerol ester[1] | 1.8 |
| Propylene glycol ester[2] | 1.8 |
| Reduced calorie fat | 54.4 |
| Milk protein (Carnation) | 3.8 |
| Powdered vanilla | 0.3 |
| Flavorings | 0.1 |

1) Average 3 glycerol units, esterified with fatty acids having from 14 to 18 carbon atoms, average 1 fatty acid molecule per glycerol unit
2) More than 65% monopalmitate ester

Potassium sorbate, xanthan gum, sucrose and citric acid are dissolved in water of 65°C in a Hobart mixer. Polyglycerol ester and propylene glycol ester are added and mixed in. The reduced calorie fat is heated to 65°C and added to the aqueous solution.

The emulsion is cooled to 26°C. At that temperature the milk protein, powdered vanilla and flavorings are mixed in.

The resulting beverage concentrate has a marshmallow cream consistency. The emulsion is mixed with milk in a 1:2.5 (w/w) ratio in a Waring blender to give a highly palatable, agreeable-tasting milkshake-like beverage.

**Claims**

1. A pharmaceutical composition in effective unit dosage amounts for inhibiting the absorption of cholesterol characterized in that it comprises from 1 to 20 grams, preferably from 2 to 10 grams, of a triglyceride fat having a fatty acid composition comprising from 5 to 70%, preferably from 10 to 50%, more preferably from 20 to 40%, $C_{20}$ to $C_{24}$ saturated fatty acids.

2. The composition of Claim 1 characterized in that said triglyceride fat is selected from the group consisting of:

(A) reduced calorie fats having:

(1) at least 80% combined MML, MLM, LLM and LML triglycerides wherein M is selected from the group consisting of $C_6$ to $C_{10}$ saturated fatty acids and mixtures thereof and L is selected from the group consisting of $C_{20}$ to $C_{24}$ saturated fatty acids and mixtures thereof, said reduced calorie fat preferably having at least 85% combined MML and MLM triglycerides, no more than 5% combined LLM and LML triglycerides, no more than about 4% MMM triglycerides, and no more than 2% LLL triglycerides, and more preferably having the following carbon number profile (CNP):

23

| CNP | (%) |
|---|---|
| 32 or lower | < 3 |
| 34 | < 2 |
| 36 | < 4 |
| 38 | 15-40 |
| 40 | 35-60 |
| 42 | 15-35 |
| 44 | < 2 |
| 46 | < 1 |
| 48 | < 0.8 |
| 50 | < 0.6 |
| 52 | < 0.4 |
| 54 or higher | < 0.9 |

(2) a fatty acid comprosition having:
   (a) from 40 to 60% $C_6$ to $C_{10}$ saturated fatty acids;
   (b) no more than 10% $C_6$ saturated fatty acid;
   (c) from 40 to 60% $C_{20}$ to $C_{24}$ saturated fatty acids;
   (d) no more than 10% $C_{20}$ saturated fatty acids;
   (e) no more than 2.5% $C_{24}$ saturated fatty acids; and
(B) hardstock fats having at least 10%, preferably from 14 to 35%, combined USS and SUS triglycerides, wherein U is selected from group consisting of $C_{18}$ to $C_{24}$ unsaturated fatty acids and mixtures thereof, preferably linoleic acid, and one S is selected from the group consisting of $C_{20}$ to $C_{24}$ saturated fatty acids and mixtures thereof, preferably behenic acid, and the other S is selected from the group consisting of $C_{18}$ to $C_{24}$ saturated fatty acids, preferably stearic acid, and mixtures thereof.

3. The composition of any of Claims 1 to 2 characterized in that it further comprises from 10 to 90%, preferably from 10 to 50%, of a pharmaceutical carrier.

4. A method for treating hypercholesterolemia characterized in that it comprises the step of systemically administering to an animal susceptible to or afflicted with hypercholesterolemia successive therapeutically effective doses of a triglyceride fat, preferably from 25 to 150 mg. per dcse, wherein the triglyceride fat has a fatty acid composition comprising from 5 to 70%, preferably from 10 to 50%, more preferably from 20 to 40%, $C_{20}$ to $C_{24}$ saturated fatty acids.

5. The method of Claim 4 characterized in that the triglyceride fat is selected from the group consisting of:
   (A) reduced calorie fats having:
      (1) at least 80% combined MML, MLM, LLM and LML triglycerides wherein M is selected from the group consisting of $C_6$ to $C_{10}$ saturated fatty acids and mixtures thereof and L is selected from the group consisting of $C_{20}$ to $C_{24}$ saturated fatty acids and mixtures thereof, the reduced calorie fat preferably having at least 85% combined MML and MLM triglycerides, no more than 5% combined LLM and LML triglycerides, no more than about 4% MMM triglycerides, and no more than 2% LLL triglycerides, and more preferably having the following carbon number profile (CNP):

24

| CNP | (%) |
|---|---|
| 32 or lower | < 3 |
| 34 | < 2 |
| 36 | < 4 |
| 38 | 15-40 |
| 40 | 35-60 |
| 42 | 15-35 |
| 44 | < 2 |
| 46 | < 1 |
| 48 | < 0.8 |
| 50 | < 0.6 |
| 52 | < 0.4 |
| 54 or higher | < 0.9 |

(2) a fatty acid comprosition having:
   (a) from 40 to 60% $C_6$ to $C_{10}$ saturated fatty acids;
   (b) no more than 10% $C_6$ saturated fatty acid;
   (c) from 40 to 60% $C_{20}$ to $C_{24}$ saturated fatty acids;
   (d) no more than 10% $C_{20}$ saturated fatty acids;
   (e) no more than 2.5% $C_{24}$ saturated fatty acids; and
(B) hardstock fats having at least 10%, preferably from 14 to 35%, combined USS and SUS triglycerides, wherein U is selected from group consisting of $C_{18}$ to $C_{24}$ unsaturated fatty acids and mixtures thereof, preferably linoleic acid, and one S is selected from the group consisting of $C_{20}$ to $C_{24}$ saturated fatty acids and mixtures thereof, preferably behenic acid, and the other S is selected from the group consisting of $C_{18}$ to $C_{24}$ saturated fatty acids, preferably stearic acid, and mixtures thereof.

6. A fat-containing food composition, characterized in that it comprises from 10 to 100%, preferably from 30 to 100%, of a triglyceride fat component comprising, in whole or in part, a triglyceride fat having:

(A) from 10 to 90%, preferably from 30 to 60%, combined MLM, MML, LLM and LML triglycerides, wherein M is selected from the group consisting of $C_6$ to $C_{10}$ saturated fatty acids and mixtures thereof, and L is selected from the group consisting of $C_{20}$ to $C_{24}$ saturated fatty acids and mixtures thereof; and

(B) from 10 to 90%, preferably from 40 to 70%, combined USS and SUS triglycerides, wherein U is selected from the group consisting of $C_{18}$ to $C_{24}$ unsaturated fatty acids and mixtures thereof, one S is selected from the group consisting of $C_{20}$ to $C_{24}$ saturated fatty acids and mixtures thereof, and the other S is selected from the group consisting of $C_{18}$ to $C_{24}$ saturated fatty acids and mixtures thereof;

wherein the fatty acid composition of said triglyceride fat component comprises from 10 to 50%, preferably from 20 to 40%, $C_{20}$ to $C_{24}$ saturated fatty acids.

7. The composition of Claim 6 characterized in that said combined MLM, MML, LLM and LML triglycerides have a fatty acid composition which comprises:
   (a) from 40 to 60% $C_6$ to $C_{10}$ saturated fatty acids;
   (b) no more than 10% $C_6$ saturated fatty acid;
   (c) from 40 to 60% $C_{20}$ to $C_{24}$ saturated fatty acids;
   (d) no more than 10% $C_{20}$ saturated fatty acid;
   (e) no more than 2.5% $C_{24}$ saturated fatty acid, and further characterized in that U is linoleic acid, one S is behenic acid and the other S is stearic acid.

8. The composition of any of Claims 6 to 7 characterized in that it is in the form of a shortening, or an emulsified spread, preferably a margarine.

9. A method for lowering cholesterol characterized in that it comprises ingesting a cholesterol-lowering amount of the composition of any of Claims 6 to 8.